# EUROPEAN PATENT SPECIFICATION

(11) **EP 2 195 646 B1**
(45) Date of publication and mention of the grant of the patent: **26.09.2012**
(21) Application number: 08836394.0
(22) Date of filing: 01.10.2008
(51) Int. Cl.: G01N 27/12, G01N 33/00, G01D 11/24

(54) **GAS MEASURING DEVICE AND METHOD OF MANUFACTURING THE SAME**
GASMESSVORRICHTUNG UND VERFAHREN ZU IHRER HERSTELLUNG
DISPOSITIF DE MESURE DE GAZ ET PROCÉDÉ D'ÉLABORATION CORRESPONDANT

(30) Priority: 01.10.2007 US 997083 P; 26.09.2008 US 238798
(43) Date of publication of application: 16.06.2010
(73) Proprietor: Scott Technologies, Inc., Monroe, NC 28110 (US)
(72) Inventor: BRISTOL, L., Rodney, Chalfont, PA 18914 (US)
(74) Representative: Hedges, Martin Nicholas
(86) International application number: PCT/US2008/011383
(87) International publication number: WO 2009/045448

(56) References cited:
- EP-A- 0 635 717
- EP-A- 1 139 099
- WO-A-2007/061294
- DE-C1- 19 638 498
- JP-A- 2003 172 719
- US-A- 5 111 792
- US-A- 5 273 779
- US-A1- 2003 019 865
- US-A1- 2004 086 023
- US-A1- 2006 117 737

## Description

The subject matter herein relates generally to gas measuring devices and methods of manufacturing gas measuring devices.

Gas measuring devices having sensors that detect certain chemicals or gases in air are utilized in many applications. For example, the detection of noxious gases such as carbon monoxide, hydrogen sulfide, nitrogen oxides, and the like is desirable so that a signal can be generated indicating the presence of such gases. Appropriate steps can then be taken to mitigate their effect or to remove persons from the presence of the gases.

One type of gas measuring device used to detect gas presence is a metal oxide semiconductor to provide early warning of the development of an explosion hazard (e.g. escaping flammable gas) or the presence of toxic gases or vapors in ambient air. The device typically includes a sensor provided on a heated substrate and includes two metallic electrodes connected to the sensor. The presence of gas posing a hazard is detected by a sensible change in the resistance of the sensor by means of the electrodes that are incorporated in a suitable electric circuit.

The reactions that allow the detection of target gases normally involve the oxidation of the target gas at the semiconductor (oxide) surface and a change in the electrical properties of the material. However, conventional sensors may be impacted by changes in temperature or humidity. Maintaining a constant temperature of the sensing element has proven problematic. At least some known devices have overcome such problems by superheating the sensor and maintaining the sensor at a superheated temperature, such that the sensor is less effected by changes in temperature. However, maintaining the sensor at superheated temperatures requires more power to operate the device.

EP1139099 describes an alcohol sensor for detecting alcohol levels in human breath. A heated sensor is provided within a housing. Internally the housing is divided into chambers in order to capture a by-pass flow from main breath flow. A gas sensor is positioned in the by-pass flow to detect alcohol. By placing the sensor in the by-pass flow rather than the main flow, cooling of the sensor is reduced.

Also, JP 2003172719 discloses a gas sensor for preventing fat and oil fire.

A need remains for a gas measuring device and sensor that may be manufactured and operated in a cost effective and reliable manner.

### BRIEF DESCRIPTION OF THE INVENTION

The present invention provides a gas measuring device according to claim 1 and a method of manufacturing a gas measuring device according to claim 8.

In one embodiment, a gas measuring device is provided for measuring a presence of a predetermined gas in a fluid medium. The gas measuring device includes a sensor case defining a sensor cavity extending from an open end of the sensor case, wherein the fluid medium flows into the sensor cavity through the open end. A sensor is positioned within the sensor cavity for sensing the presence of the predetermined gas, wherein the sensor has a sensing element on one side of the sensor, and the sensing element is positioned within the sensor cavity such that the sensing element faces away from the opening.

Optionally, a porous screen may cover the open end and allow the fluid medium to flow therethrough. The sensor case may include a bottom generally opposed to the open end, and the sensor is positioned within the sensor cavity such that the sensing element faces the bottom of the sensor case. The sensing element may be responsive to a predetermined gas such that the electrical properties of the sensing element vary based on a presence of the predetermined gas. Optionally, the gas measuring device may further include a controller operatively connected to the sensing element for measuring at least one electrical property of the sensing element. The sensor may include a substrate having the sensing element applied to one side of the substrate and a heating element applied to the opposite side of the substrate, wherein the heating element is capable of heating the sensing element to a predetermined temperature. The sensing element and the heating element may cover substantially similar areas of the substrate.

In another embodiment, a method of manufacturing a gas measuring device is provided wherein the method includes providing a sensor case defining a sensor cavity extending from an open end of the sensor case and optionally covering the open end of the sensor cavity with a porous screen. The method also includes positioning a sensor having a sensing element on one side of the sensor within the sensor cavity such that the sensing element faces away from the opening or optional screen. The sensor is configured for sensing a presence of a target gas flowing into the sensor cavity.

In a further embodiment, a method of manufacturing a gas sensor is provided that includes providing a substrate having a sensing element and a heating element. The heating element is capable of heating the sensing element to an operating temperature, and the heating element has a resistance characteristic. The method also includes calibrating the heating element by measuring an initial resistance of the heating element at a first temperature less than the operating temperature of the gas sensor, and then calculating an operating resistance of the heating element at the operating temperature.

### BRIEF DESCRIPTION OF THE DRAWINGS

Figure 1 illustrates a gas measuring device having a sensor formed in accordance with an exemplary embodiment.
Figure 2 illustrates a control circuit for the gas measuring device and sensor shown in Figure 1.
Figure 3 is a cross sectional view of an exemplary embodiment of the sensor for the gas measuring device shown in Figure 1.
Figure 4 is a flow chart for an exemplary method of manufacturing a gas measuring device, such as the gas measuring device shown in Figure 1.
Figure 5 is a flow chart for another exemplary method of manufacturing a gas measuring device, such as the gas measuring device shown in Figure 1.

### DETAILED DESCRIPTION OF THE INVENTION

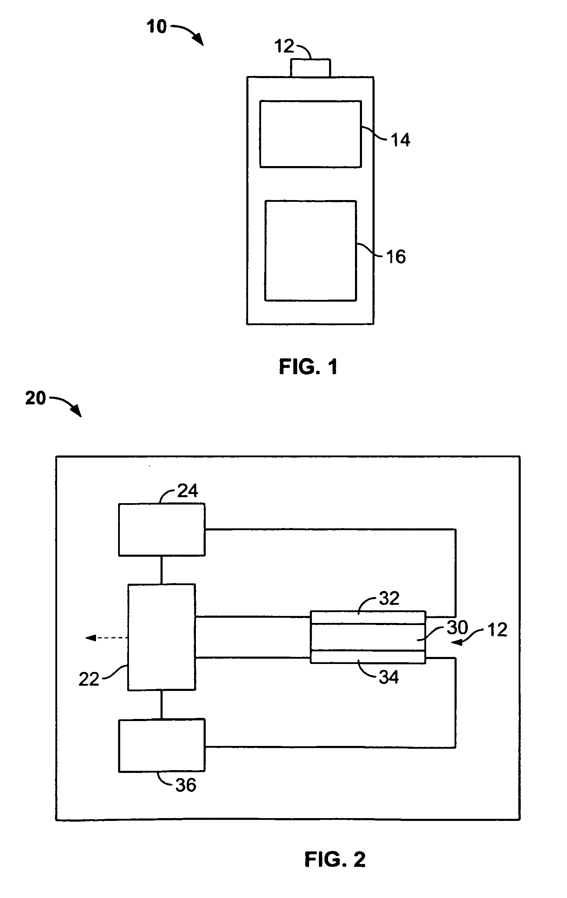

Figure 1 illustrates a gas measuring device 10 having a sensor 12. The gas measuring device 10 is used for measuring a presence of a certain gas or gases in a fluid medium, such as air. The gas measuring device 10 may be used for measuring the presence of noxious, toxic, combustible or other harmful type of gas that may be present in the fluid medium. For example, the gas measuring device 10 may be used to detect for gases such as hydrogen sulfide, carbon monoxide, nitrogen oxides, and the like. In one embodiment, the gas measuring device 10 merely detects whether or not the gas is present. In other embodiments, the gas measuring device 10 may detect the amount or concentration of the gas when present.

The gas measuring device 10 communicates results, such as the concentration of the gas, by any known method, such as on a display 14. Optionally, a user interface 16, such as a keypad, may be provided for user interaction with the device 10. The gas measuring device 10 may alert the user as to the presence of a gas, or the presence of a gas above a threshold level, such as with an audible or visible alarm. In some embodiments, the gas measuring device 10 may communicate with other devices or systems to alert the device or system as to the presence of a gas. Such communication may be wired or wireless. The gas measuring device 10 may be portable and carried by a user, or alternatively, may be mounted to a structure in a desired location.

The sensor 12 is provided on the gas measuring device 10 such that the sensor 12 is exposed to the air surrounding the gas measuring device 10. While the sensor 12 is illustrated as being provided on an external surface of the gas measuring device 10, the sensor 12 may be internally housed within the gas measuring device 10 and air flow may be directed to the sensor 12, such as through a port open to the external environment or by air being pumped to the sensor 12.

Figure 2 schematically illustrates a control circuit 20 for the gas measuring device 10 and sensor 12. The control circuit 20 includes a controller 22 that is operatively connected to the sensor 12. The control circuit 20 also includes a low voltage power supply 24 that is operatively connected to the sensor 12.

In an exemplary embodiment, the sensor 12 is a metal oxide sensor, however, other types of sensors may be used with the gas measuring device 10, and the metal oxide sensor illustrated in the figures is illustrative and is not intended to be limiting. The sensor 12 includes a substrate 30, such as a ceramic insulator. One example of a substrate 30 for the sensor 12 is an alumina substrate. The sensor 12 also includes a sensing element 32 on the substrate 30 and a heating element 34 on the substrate 30. In an exemplary embodiment, the sensing element 32 includes a gas sensitive material and is applied to one side of the substrate 30. The heating element 34 is applied to the opposite side of the substrate 30. In an alternative embodiment, the sensing element 32 and the heating element 34 may be applied to the same side of the substrate 30. Optionally, the sensor 12 may be a layered structure, wherein the sensing element 32 and/or the heating element 34 are layers applied to the substrate 30. For example, the sensing element 32 may be a film material screen printed on the substrate 30. The sensing element 32 may be a porous nanostructure. Similarly, the heating element 34 may be a film material screen printed on the substrate 30. The heating element 34 may be a ceramic micromachined heater.

The low voltage power supply 24 is operatively connected to the sensing element 32 and supplies a predetermined voltage to the sensing element 32. Optionally, the voltage supplied may be a constant voltage. The controller 22 is also connected to the sensing element 32 and measures at least one electrical property of the sensing element 32, such as a resistance, a conductance, a capacitance, and/or an impedance. The sensing element 32 is manufactured using a material having electrical properties that are affected by the presence of a predetermined target gas. For example, the sensing element 32 may be responsive to the gas such that the electrical properties of the sensing element 32 vary based on the presence and/or concentration of the gas. In an exemplary embodiment, gas adsorption on the surface of the sensing element 32 causes a change in electrical properties of the sensing element 32, such as a change in resistance. The changes in the electrical properties are detected and/or measured by the controller 22.

In an exemplary embodiment, the gas reactions with the sensing element 32 occur when the sensing element 32 is at an elevated temperature. The heating element 34 is used to elevate the temperature of the sensing element 32 to a predetermined temperature. A heater power supply 36 is provided for supplying power to the heating element 34. The power supply 36 may be operatively controlled by the controller 22, for example by pulse modulation. The amount of power supplied, the duration of the pulse and the frequency of the pulses affect the temperature of the heating element 34, and thus the sensing element 32. For example, in operation, when the heating element 34 is powered, the temperature of the substrate 30 is elevated, which thus raises the temperature of the sensing element 32 to a predetermined level. When the temperature of the sensing element 32 is at a predetermined level, the sensing element 32 may react with the gas at the surface of the sensing element 32. In an exemplary embodiment, the controller 22 is also connected to the heating element 34 to measure an electrical property of the heating element 34, such as a resistance, a conductance, a capacitance, and/or an impedance. The power supply to the heating element 34 may be controlled based on the electrical properties of the heating element 34. For example, the temperature of the heating element 34 may be related to a measured property of the heating element 34, such as resistance. As such, the temperature of the heating element 34 may be calculated and/or changed based on a measured electrical property of the heating element 34, such as the resistance. The gas measuring device 10 may be manufactured and operated in a similar manner as the device and sensor illustrated and described in copending U.S. Patent Application titled "GAS MEASURING DEVICE AND METHOD OF OPERATING THE SAME", filed concurrently herewith and incorporated by reference herein in its entirety.

In alternative embodiments, other control schemes other than pulse modulation may be implemented to control the power supply to the heating element 34. In another embodiment, the heater power supply voltage may be proportionally controlled to maintain a desired heater resistance, where heater resistance is calculated from the ratio of heater voltage and a measurement of heater current.

In operation, when the controller 22 detects the presence of the gas, the controller 22 may output a signal relating to such presence and/or the concentration of the gas. The signal output from the controller 22 may be used by the gas measuring device 10 to alert the user and/or to display information relating to the presence/concentration of gas. In an exemplary embodiment, the controller 22 may include circuitry or circuit components, such as an amplifier that manipulates the signal from the sensing element 32, and/or an analog-to-digital converter that manipulates the signal from the sensing element 32. The manipulated signal may be output from the controller 22, or otherwise used by the gas measuring device 10 to perform other functions of the gas measuring device 10, such as the alerting or displaying. While the controller 22 is illustrated as being a common controller 22 that is operatively connected to both the heating element 34 and the sensing element 32, the control circuit 20 may include more than one controller.

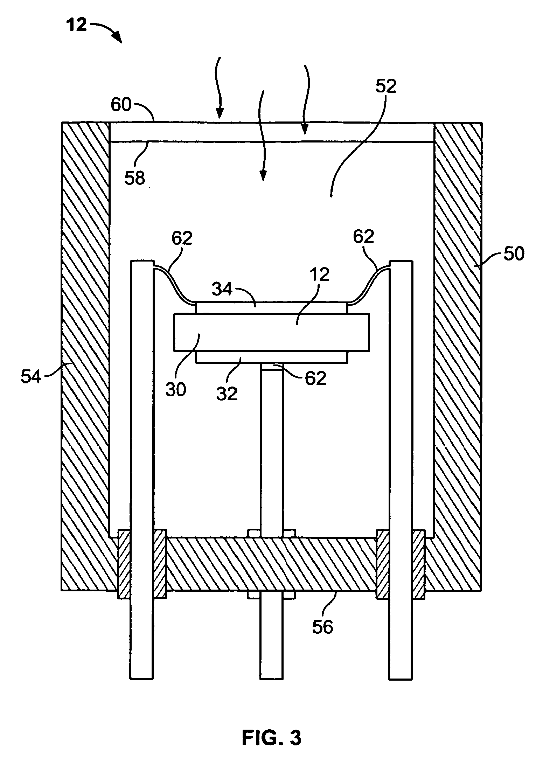

Figure 3 is a cross sectional view of an exemplary embodiment of the sensor 12 for the gas measuring device 10 (shown in Figure 1). The sensor 12 includes a sensor case 50 defining a sensor cavity 52. The sensor case 50 includes side walls 54 and a closed bottom 56. The sensor case 50 also includes an open top 58, generally opposite the bottom 56, and, optionally, a filter or screen element 60 at the open top 58. The screen element 60 is porous and allows air flow therethrough. Optionally, the screen element 60 may be used to filter certain gases from flowing therethrough. In an exemplary embodiment, the sensor case 50 may be generally cylindrical, but other shapes are possible in alternative embodiments.

The sensor 12 is positioned within the sensor cavity 52 for sensing the presence of predetermined target gases in the air flowing through the sensor cavity 52. Air from the external environment is able to flow through the screen element 60 and around the sensor 12. In an exemplary embodiment, the sensor 12 is positioned within the sensor cavity 52 such that the sensing element 32 faces away from the open top 58. For example, the sensing element 32 may generally face the bottom 56. Because the ambient environment is at a lower temperature than the sensor, and the open top 58 or, if it is used, the screen element 60, has a higher emissivity as compared to the sensor case 50, positioning the sensing element 32 away from the opening or the screen element 60 may reduce the rate or amount of reduction in temperature of the sensing element 32. As such, the temperature of the sensing element 32 may be more easily maintained at a constant temperature as compared to having the sensing element 32 face the ambient environment or the optional screen element 60. The sensor 12 may be positioned closer to the bottom 56 than the top 58 such that the sensing element 32 is proximate to the bottom 56 to efficiently retain the heat and thus maintain the temperature.

In an exemplary embodiment, the sensing element 32 and the heating element 34 each cover a predetermined amount of the substrate 30. Optionally, the sensing element 32 and the heating element 34 may cover substantially all of the substrate 30, however, the surface area of the sensing element 32 and the heating element 34 may be selected to cover only a select portion of the substrate 30. In one embodiment, the surface areas of the sensing element 32 and the heating element 34 may have substantially similar footprints (e.g. be substantially identically sized and shaped) and may be aligned with one another. As such, the heating element 34 may uniformly heat the sensing element 32. Optionally, the heating element 34 may have a larger footprint than the sensing element 32 such that the heating element 34 entirely covers the sensing element 32. For example, the perimeter of the sensing element 32 fits within the perimeter of the heating element 34.

A plurality of electrodes 62 are connected to the sensor 12. The electrodes 62 extend through the sensor case 50 into the sensor cavity 52. In an exemplary embodiment, the electrodes 62 are part of the control circuit 20 and connect the sensor 12 with the controller 22 (shown in Figure 2) and/or the power supplies 24 and 36. Optionally, some electrodes 62 are connected to the heating element 34 and some electrodes 62 are connected to the sensing element 32.

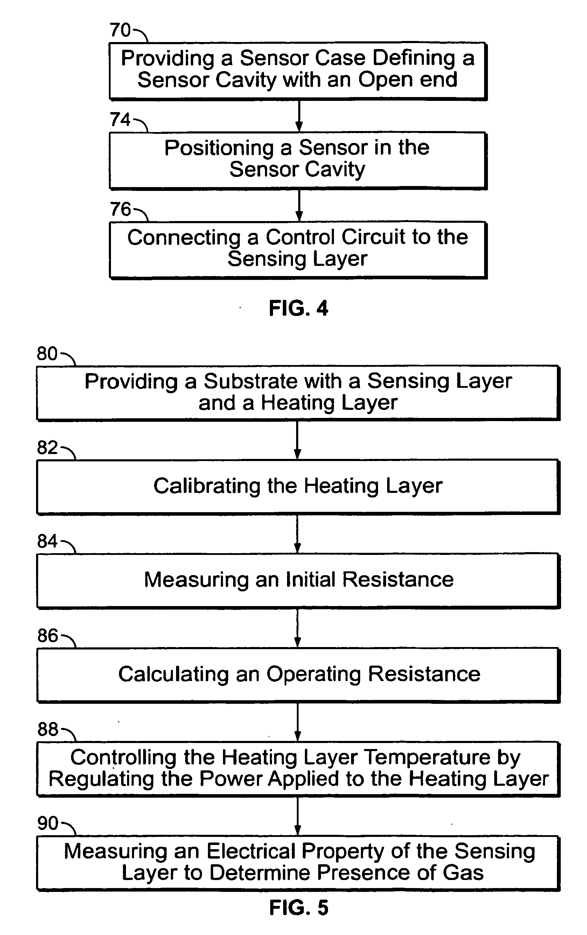

Figure 4 is a flow chart illustrating an exemplary method of manufacturing a gas measuring device, such as the gas measuring device 10 (shown in Figure 1). The method includes providing 70 a sensor case defining a sensor cavity extending from an open end of the sensor case. Optionally, the open end of the sensor cavity may be covered with a porous screen. The method also includes positioning 74 a sensor within the sensor cavity. The sensor may have a sensing element on one side of the sensor such that the sensing element faces away from the screen, and the sensor may be configured for sensing a presence of a predetermined target gas flowing into the sensor cavity through the porous screen. Other sensor types may be provided in alternative embodiments.

The sensor may be positioned within the sensor cavity such that the sensing element faces a closed bottom of the sensor case that is generally opposed to the open face. The sensor may have a sensing element that is responsive to a predetermined target gas such that the electrical properties of the sensing element vary based on a presence of the predetermined target gas. The sensor may include a substrate having the sensing element applied to one side of the substrate and a heating element applied to the opposite side of the substrate, wherein the heating element is capable of heating the sensing element to a predetermined temperature.

The method may also include the step of connecting 76 a controller to the sensing element for measuring at least one electrical property of the sensing element. In operation, the controller is used to monitor or measure at least one electrical property of the sensing element to detect the presence of at least one gas of interest in the air. In operation, the controller is also used to control the temperature of the heating element. For example, the controller may include a heater power supply that is operatively coupled to the heating element. The controller supplies power to the heating element according to a control scheme for elevating the temperature of the heating element and the sensing element. Optionally, the controller may monitor or measure at least one electrical property of the heating element to control the power supply to the heating element.

Figure 5 is a flow chart illustrating another exemplary method of manufacturing a gas measuring device, such as the gas measuring device 10 (shown in Figure 1). The method includes providing 80 a substrate having a sensing element and a heating element, wherein the heating element is capable of heating the sensing element to a predetermined operating temperature and wherein the heating element has a resistance characteristic that may be different from one sensor to another. The resistance characteristic may vary from one sensor to another as directly or indirectly affected by such properties as thickness, bulk resistivity, and surface area of the sensing element. Rather than tightly controlling the resistance characteristic, such as by trimming the sensing element or controlling the size of the sensing element by a costly application or manufacturing step, the system is configured to accommodate variations in resistance characteristics between successive devices by calibrating the device to the particular sensor. The method includes calibrating 82 the heating element by measuring 84 an initial resistance of the heating element at a first temperature less than the operating temperature of the gas sensor, and then calculating 86 an operating resistance of the heating element at the operating temperature. In common practice according to prior art, an operating temperature of the sensing element is approximately determined by providing a predetermined heating element resistance and connecting a predetermined power supply operated at a predetermined operating point, such as 28 Ohms, to the heating element. Tightly controlling properties to achieve a predetermined resistance may incur significant costs in fabrication. Additionally, trimming the heating element to obtain a predetermined resistance may impair the thermal coupling between the heating element and sensing element, which may impair the accuracy of the temperature of the sensing element. For example, the sensing element may have a different shape than the heating element after trimming which may affect the thermal gradient of the sensing element, such as heating the sensing element non-uniformly across the surface area thereof.

The method may include the step of connecting the heating element to a controller and controlling 88 the heating element temperature by regulating the power applied to the heating element as needed to maintain the calculated operating temperature. The controller measures a resistance of the heating element and the controller is configured to electrically power the heating element to increase the temperature of the heating element until the resistance is at the operating resistance. The controller may control the temperature of the heating element. The method may also include the step of connecting the sensing element to a controller and measuring 90 an electrical property of the sensing element to determine the presence of a target gas. The sensing element is responsive to a predetermined target gas such that the electrical properties of the sensing element vary based on a presence of the predetermined gas. The controller may measure the electrical properties of the sensing element.

During operation of the gas sensor, an electrical property of the heating element, such as the resistance, is measured by the controller. The resistance of the heating element may be affected by the size, surface area, and/or volume of the heating element, and as such, the gas sensor may need to be calibrated as the size, surface area, and/or volume of the heating element may be different for different gas sensors. The electrical properties of the heating element are also affected by the temperature of the heating element, and as such, the gas sensor may have a desired operating temperature. In an exemplary embodiment, the heating element has a given electrical property characteristic that can be used to compare the electrical property versus the temperature. For example, the characteristic may be a substantially linear characteristic, a non-linear characteristic, a substantially logarithmic characteristic, and the like. When the temperature is increased, the resistance may vary according to the characteristic, and similarly, when the temperature is decreased, the resistance may vary according to the characteristic. As such, when the initial resistance at a known initial temperature (e.g. room temperature) is measured, the operating resistance for the heating element may be calculated based on a desired operating temperature. For example, if the desired operating temperature of the heating element is 350 degrees, then the operating resistance may be calculated based on the characteristic of resistance versus temperature. As such, during operation, the temperature of the heating element may be controlled by using the controller to measure the resistance of the heating element, and either increasing or decreasing the power applied to the heating element based on the measured resistance.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. Dimensions, types of materials, orientations of the various components, and the number and positions of the various components described herein are intended to define parameters of certain embodiments, and are by no means limiting and are merely exemplary embodiments. Many other embodiments and modifications within the spirit and scope of the claims will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A gas measuring device for measuring a presence of a target gas in a fluid medium, the gas measuring device (10) comprising:
a sensor case (50) having closed a bottom (56) and defining a sensor cavity (52) extending from an open end (58) of the sensor case (50) with the open end (58) being opposed to the bottom (56), wherein the fluid medium flows into the sensor cavity (52) through the open end (58); and
a sensor (12) positioned within the sensor cavity (52) for sensing the presence of the target gas, the sensor (12) comprising a substrate (30) and a sensing element (32) on the substrate (30), the gas measuring device being **characterised by**
the sensor (12) being positioned within the sensor cavity (52) such that the substrate (30) is disposed between the sensing element (32) and the open end (58) with the sensing element (32) adjacent to the bottom (56) of the cavity (52).

2. The gas measuring device of claim 1, wherein the sensing element (32) is responsive to a target gas such that the electrical properties of the sensing element (32) vary based on a presence of the target gas.

3. The gas measuring device of claim 1, further comprising a circuit (20) operatively connected to the sensing element (32) for measuring at least one electrical property of the sensing element (32).

4. The gas measuring device of claim 1, wherein the sensing element (32) is applied to one side of the substrate (30) and a heating element (34) is applied to the opposite side of the substrate (30), the heating element (34) being capable of heating the sensing element (32) to a predetermined temperature.

5. The gas measuring device of claim 1, wherein the sensing element (32) is applied to one side of the substrate (30) and a heating element (34) is applied to the opposite side of the substrate (30), the sensing element (32) and the heating element (34) covering substantially similar areas of the substrate (30).

6. The gas measuring device of claim 1, wherein the sensing element (32) is applied to one side of the substrate (30) and a heating element (34) is applied to the opposite side of the substrate (30), the heating element (34) covering at least as much of the substrate (30) as the sensing element (32).

7. The gas measuring device of claim 1, wherein the sensing element (32) and the heating element (34) are aligned with one another on opposite sides of the substrate (30) such that a perimeter of the sensing element (32) fits within a perimeter of the heating element (34).

8. A method of manufacturing a gas measuring device comprising:
providing a sensor case (50) with a closed bottom (56), the sensor case (50) defining a sensor cavity (52) extending from an open end (58) of the sensor case (50) with the open end opposed to the bottom (56); and
positioning a sensor (12), comprising a substrate (30) and a sensing element (32) on the substrate (30), within the sensor cavity (52), wherein the sensor (12) is configured for sensing a presence of a target gas flowing into the sensor cavity (52), the method being **characterised by** the method step of
positioning the sensor (12) within the sensor cavity (52) such that the sensing element (32) is adjacent to the bottom (56) of the cavity (52) with the substrate (30) disposed between the sensing element (32) and the open end of the cavity (58).

9. The method of claim 8, wherein the positioning further comprises positioning a sensor (12) having the sensing element (32) applied to one side of the substrate (30) and a heating element (34) applied to the opposite side of the substrate (30), the heating element (34) being capable of heating the sensing element (32) to a predetermined temperature.

10. The method of claim 8, wherein the positioning further comprises positioning a sensor (12) having the sensing element (32) applied to one side of the substrate (30) and a heating element (34) applied to the opposite side of the substrate (30), wherein the sensing element (32) and the heating element (34) cover substantially similar areas of the substrate (30).

11. The method of claims 9 or10, further comprising:
calibrating the heating element (34) by measuring an initial resistance of the heating element (34) at a first temperature less than the operating temperature of the gas sensor (12), and then calculating an operating resistance of the heating element (34) at the operating temperature.

12. The method of claim 11, wherein the heating element (34) has a surface area and the resistance characteristic is based on the surface area, the calibrating being performed to allow for variations in the surface area of the heating element (34).

13. The method of claim 11, wherein the operating resistance of the sensing element (32) is determined with respect to its resistance characteristics.

14. The method of claim 11, wherein, the calculated operating resistance of the heating element (34) is dependent on the measured initial resistance of the heating element (34).15.

15. The method of claim 11, further comprising connecting the heating element (34) to a controller (22), wherein the controller (22) measures a resistance of the heating element (34) and the controller (22) is configured to electrically power the heating element (34) to increase the temperature of the heating element (34) until the resistance is at the operating resistance.

16. The method of claim 11, further comprising controlling the heating element (34) temperature by regulating the power applied to the heating element (34) as needed to maintain the calculated operating temperature.

17. The method of claim 11, further comprising connecting the sensing element (32) to a controller (22), and measuring a property of the sensing element (32) to determine the presence of a target gas.

18. The method of claim 11, further comprising connecting the sensing element (32) to a controller (22), wherein the sensing element (32) is responsive to a target gas such that the electrical properties of the sensing element (32) vary based on a presence of the target gas, and wherein the controller measures the electrical properties of the sensing element (32).

## Patentansprüche

1. Gasmessvorrichtung zum Messen eines Vorhandenseins eines Zielgases in einem Fluidmedium, die Gasmessvorrichtung (10) umfassend:
ein Sensorgehäuse (50) mit einem geschlossenen Boden (56) und definierend einen Sensorhohlraum (52), der sich von einem offenen Ende (58) des Sensorgehäuses (50) erstreckt, wobei das offene Ende (58) dem Boden (56) gegenüberliegt, wobei das Fluidmedium durch das offene Ende (58) in den Sensorhohlraum (52) strömt; und
einen Sensor (12), positioniert innerhalb des Sensorhohlraums (52), zum Erfassen des Vorhandenseins des Zielgases, der Sensor (12) umfassend ein Substrat (30) und ein Erfassungselement (32) auf dem Substrat (30), die Gasmessvorrichtung **gekennzeichnet dadurch, dass**
der Sensor (12) derart innerhalb des Sensorhohlraums (52) positioniert ist, dass das Substrat (30) zwischen dem Erfassungselement (32) und dem offenen Ende (58) mit dem Erfassungselement (32) angrenzend an dem Boden (56) des Hohlraums (52) angeordnet ist.

2. Gasmessvorrichtung nach Anspruch 1, wobei das Erfassungselement (32) derart auf ein Zielgas reagiert, dass die elektrischen Eigenschaften des Erfassungselements (32) basierend auf einem Vorhandensein des Zielgases variieren.

3. Gasmessvorrichtung nach Anspruch 1, weiter umfassend eine Schaltung (20), die betriebsfähig mit dem Erfassungselement (32) zum Messen mindestens einer elektrischen Eigenschaft des Erfassungselements (32) verbunden ist.

4. Gasmessvorrichtung nach Anspruch 1, wobei das Erfassungselement (32) auf einer Seite des Substrats (30) aufgebracht ist und ein Heizelement (34) auf der gegenüberliegenden Seite des Substrats (30) aufgebracht ist, wobei das Heizelement (34) imstande ist, das Erfassungselement (32) auf eine im Voraus bestimmte Temperatur zu erwärmen.

5. Gasmessvorrichtung nach Anspruch 1, wobei das Erfassungselement (32) auf einer Seite des Substrats (30) aufgebracht ist und ein Heizelement (34) auf der gegenüberliegenden Seite des Substrats (30) aufgebracht ist, wobei das Erfassungselement (32) und das Heizelement (34) im Wesentlichen ähnliche Bereiche des Substrats (30) bedecken.

6. Gasmessvorrichtung nach Anspruch 1, wobei das Erfassungselement (32) auf einer Seite des Substrats (30) aufgebracht ist und ein Heizelement (34) auf der gegenüberliegenden Seite des Substrats (30) aufgebracht ist, wobei das Heizelement (34) mindestens so viel des Substrats (30) wie das Erfassungselement (32) bedeckt.

7. Gasmessvorrichtung nach Anspruch 1, wobei das Erfassungselement (32) und das Heizelement (34) auf gegenüberliegenden Seiten des Substrats (30) derart miteinander ausgerichtet sind, dass ein Umfang des Erfassungselements (32) innerhalb eines Umfangs des Heizelements (34) passt.

8. Verfahren zur Herstellung einer Gasmessvorrichtung, umfassend:
Bereitstellen eines Sensorgehäuses (50) mit einem geschlossenen Boden (56), wobei das Sensorgehäuse (50) einen Sensorhohlraum (52) definiert, der sich von einem offenen Ende (58) des Sensorgehäuses (50) erstreckt, wobei das offene Ende dem Boden (56) gegenüberliegt; und
Positionieren eines Sensors (12), der ein Substrat (30) und ein Erfassungselement (32) auf dem Substrat (30) umfasst, innerhalb des Sensorhohlraums (52), wobei der Sensor (12) konfiguriert ist zum Erfassen des Vorhandenseins eines Zielgases, das in den Sensorhohlraum (52) strömt, das Verfahren **gekennzeichnet durch** den folgenden Verfahrensschritt
Positionieren des Sensors (12) derart innerhalb des Sensorhohlraums (52), dass das Erfassungselement (32) angrenzend an dem Boden (56) des Hohlraums (52) mit dem Substrat (30) zwischen dem Erfassungselement (32) und dem offenen Ende des Hohlraums (58) angeordnet ist.

9. Verfahren nach Anspruch 8, wobei das Positionieren weiter umfasst, einen Sensor (12) mit dem Erfassungselement (32) auf einer Seite des Substrats (30) aufgebracht und einem Heizelement (34) auf der gegenüberliegenden Seite des Substrats (30) aufgebracht zu positionieren, wobei das Heizelement (34) imstande ist, das Erfassungselement (32) auf eine im Voraus bestimmte Temperatur zu erwärmen.

10. Verfahren nach Anspruch 8, wobei das Positionieren weiter umfasst, einen Sensor (12) mit dem Erfassungselement (32) auf einer Seite des Substrats (30) aufgebracht und einem Heizelement (34) auf der gegenüberliegenden Seite des Substrats (30) aufgebracht zu positionieren, wobei das Erfassungselement (32) und das Heizelement (34) im Wesentlichen ähnliche Bereiche des Substrats (30) bedecken.

11. Verfahren nach Anspruch 9 oder 10, weiter umfassend:
Kalibrieren des Heizelements (34) durch Messen eines anfänglichen Widerstands des Heizelements (34) bei einer ersten Temperatur, die geringer ist als die Betriebstemperatur des Gassensors (12), und dann Berechnen eines Betriebswiderstands des Heizelements (34) bei der Betriebstemperatur.

12. Verfahren nach Anspruch 11, wobei das Heizelement (34) einen Oberflächenbereich aufweist und die Widerstandscharakteristik auf dem Oberflächenbereich basiert, wobei das Kalibrieren durchgeführt wird, um Variationen im Oberflächenbereich des Heizelements (34) zuzulassen.

13. Verfahren nach Anspruch 11, wobei der Betriebswiderstand des Erfassungselements (32) in Bezug auf seine Widerstandscharakteristik bestimmt wird.

14. Verfahren nach Anspruch 11, wobei der berechnete Betriebswiderstand des Heizelements (34) von dem gemessenen Anfangswiderstand des Heizelements (34) abhängig ist.

15. Verfahren nach Anspruch 11, weiter umfassend, das Heizelement (34) mit einer Steuerung (22) zu verbinden, wobei die Steuerung (22) einen Widerstand des Heizelements (34) misst und die Steuerung (22) konfiguriert ist, um das Heizelement (34) elektrisch anzutreiben, um die Temperatur des Heizelements (34) zu erhöhen, bis der Widerstand an dem Betriebswiderstand ist.

16. Verfahren nach Anspruch 11, weiter umfassend, die Temperatur des Heizelements (34) durch Regeln der dem Heizelement (34) zugeführten Leistung nach Erfordernis, um die berechnete Betriebstemperatur beizubehalten, zu steuern.

17. Verfahren nach Anspruch 11, weiter umfassend, das Erfassungselement (32) mit einer Steuerung (22) zu verbinden und eine Eigenschaft des Erfassungselements (32) zu messen, um das Vorhandensein eines Zielgases zu bestimmen.

18. Verfahren nach Anspruch 11, weiter umfassend, das Erfassungselement (32) mit einer Steuerung (22) zu verbinden, wobei das Erfassungselement (32) derart auf ein Zielgas reagiert, dass die elektrischen Eigenschaften des Erfassungselements (32) basierend auf einem Vorhandensein des Zielgases variieren, und wobei die Steuerung die elektrischen Eigenschaften des Erfassungselements (32) misst.

## Revendications

1. Dispositif de mesure de gaz pour mesurer la présence d'un gaz cible dans un milieu fluide, le dispositif de mesure de gaz (10) comprenant :
un boîtier de capteur (50) ayant un fond fermé (56) et définissant une cavité de capteur (52) s'étendant depuis une extrémité ouverte (58) du boîtier de capteur (50), l'extrémité ouverte (58) étant opposée au fond (56), le milieu fluide s'écoulant dans la cavité de capteur (52) par l'extrémité ouverte (58) ; et
un capteur (12) positionné à l'intérieur de la cavité de capteur (52) pour détecter la présence du gaz cible, le capteur (12) comprenant un substrat (30) et un élément de détection (32) sur le substrat (30), le dispositif de mesure de gaz étant **caractérisé par le fait que**
le capteur (12) est positionné à l'intérieur de la cavité de capteur (52) de telle sorte que le substrat (30) soit disposé entre l'élément de détection (32) et l'extrémité ouverte (58) avec l'élément de détection (32) adjacent au fond (56) de la cavité (52).

2. Dispositif de mesure de gaz selon la revendication 1, dans lequel l'élément de détection (32) est sensible à un gaz cible de telle sorte que les propriétés électriques de l'élément de détection (32) varient en fonction de la présence du gaz cible.

3. Dispositif de mesure de gaz selon la revendication 1, comprenant en outre un circuit (20) connecté opérationnellement à l'élément de détection (32) pour mesurer au moins une propriété électrique de l'élément de détection (32).

4. Dispositif de mesure de gaz selon la revendication 1, dans lequel l'élément de détection (32) est appliqué sur un côté du substrat (30) et un élément chauffant (34) est appliqué sur le côté opposé du substrat (30), l'élément chauffant (34) étant capable de chauffer l'élément de détection (32) à une température prédéterminée.

5. Dispositif de mesure de gaz selon la revendication 1, dans lequel l'élément de détection (32) est appliqué sur un côté du substrat (30) et un élément chauffant (34) est appliqué sur le côté opposé du substrat (30), l'élément de détection (32) et l'élément chauffant (34) couvrant sensiblement des parties similaires du substrat (30).

6. Dispositif de mesure de gaz selon la revendication 1, dans lequel l'élément de détection (32) est appliqué sur un côté du substrat (30) et un élément chauffant (34) est appliqué sur le côté opposé du substrat (30), l'élément chauffant (34) couvrant au moins autant du substrat (30) que l'élément de détection (32).

7. Dispositif de mesure de gaz selon la revendication 1, dans lequel l'élément de détection (32) et l'élément chauffant (34) sont alignés l'un avec l'autre sur des côtés opposés du substrat (30) de telle sorte qu'un périmètre de l'élément de détection (32) entre dans un périmètre de l'élément chauffant (34).

8. Procédé de fabrication d'un dispositif de mesure de gaz comprenant :
la fourniture d'un boîtier de capteur (50) ayant un fond fermé (56), le boîtier de capteur (50) définissant une cavité de capteur (52) s'étendant depuis une extrémité ouverte (58) du boîtier de capteur (50), l'extrémité ouverte (58) étant opposée au fond (56) ; et
le positionnement d'un capteur (12), comprenant un substrat (30) et un élément de détection (32) sur le substrat (30), à l'intérieur de la cavité de capteur (52), le capteur (12) étant configuré pour détecter la présence d'un gaz cible s'écoulant dans la cavité de capteur (52), le procédé étant **caractérisé par** l'étape de procédé de
le positionnement du capteur (12) à l'intérieur de la cavité de capteur (52) de telle sorte que le l'élément de détection (32) soit adjacent au fond (56) de la cavité (52), le substrat (30) étant disposé entre l'élément de détection (32) et l'extrémité ouverte (58) de la cavité.

9. Procédé selon la revendication 8, dans lequel le positionnement comprend en outre le positionnement d'un capteur (12) comportant l'élément de détection (32) appliqué sur un côté du substrat (30) et un élément chauffant (34) appliqué sur le côté opposé du substrat (30), l'élément chauffant (34) étant capable de chauffer l'élément de détection (32) à une température prédéterminée.

10. Procédé selon la revendication 8, dans lequel le positionnement comprend en outre le positionnement d'un capteur (12) comportant l'élément de détection (32) appliqué sur un côté du substrat (30) et un élément chauffant (34) appliqué sur le côté opposé du substrat (30), l'élément de détection (32) et l'élément chauffant (34) couvrant sensiblement des zones similaires du substrat (30).

11. Procédé selon la revendication 9 ou 10, comprenant en outre :
l'étalonnage de l'élément chauffant (34) en mesurant une résistance initiale de l'élément chauffant (34) à une première température inférieure à la température opérationnelle du capteur de gaz (12), puis en calculant une résistance opérationnelle de l'élément chauffant (34) à la température opérationnelle.

12. Procédé selon la revendication 11, dans lequel l'élément chauffant (34) a une superficie et la caractéristique de résistance est basée sur la superficie, l'étalonnage étant exécuté pour permettre des variations de la superficie de l'élément chauffant (34).

13. Procédé selon la revendication 11, dans lequel la résistance opérationnelle de l'élément de détection (32) est déterminée relativement à sa caractéristique de résistance.

14. Procédé selon la revendication 11, dans lequel la résistance opérationnelle calculée de l'élément chauffant (34) dépend de la résistance initiale mesurée de l'élément chauffant (34).

15. Procédé selon la revendication 11, comprenant en outre la connexion de l'élément chauffant (34) à une unité de commande (22), l'unité de commande (22) mesurant une résistance de l'élément chauffant (34) et l'unité de commande (22) étant configurée pour alimenter électriquement l'élément chauffant (34) afin d'augmenter la température de l'élément chauffant (34) jusqu'à ce que la résistance soit à la résistance opérationnelle.

16. Procédé selon la revendication 11, comprenant en outre la commande de la température de l'élément chauffant (34) en régulant la puissance appliquée à l'élément chauffant (34) comme il convient pour maintenir la température opérationnelle calculée.

17. Procédé selon la revendication 11, comprenant en outre la connexion de l'élément de détection (32) à une unité de commande (22), et la mesure d'une propriété de l'élément de détection (32) pour déterminer la présence d'un gaz cible.

18. Procédé selon la revendication 11, comprenant en outre la connexion de l'élément de détection (32) à une unité de commande (22), l'élément de détection (32) étant sensible à un gaz cible de telle sorte que les propriétés électriques de l'élément de détection (32) varient en fonction de la présence du gaz cible, et l'unité de commande mesurant les propriétés électriques de l'élément de détection (32).
